(19) **Europäisches Patentamt / European Patent Office / Office européen des brevets**

(11) **EP 4 394 042 A1**

(12) **EUROPEAN PATENT APPLICATION**
published in accordance with Art. 153(4) EPC

(43) Date of publication:
**03.07.2024 Bulletin 2024/27**

(21) Application number: **22860105.0**

(22) Date of filing: **15.07.2022**

(51) International Patent Classification (IPC):
*C12N 15/85* (2006.01)    *C07K 19/00* (2006.01)
*C12N 15/62* (2006.01)    *A61K 38/20* (2006.01)
*A61K 47/64* (2017.01)    *A61P 25/14* (2006.01)

(86) International application number:
**PCT/CN2022/106115**

(87) International publication number:
**WO 2023/024759 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.08.2021 CN 202110982310**

(71) Applicant: **Beijing Vdjbio Co., Ltd.
Beijing 100085 (CN)**

(72) Inventors:
• **ZHANG, Xiaorui
Beijing 100085 (CN)**
• **CHENG, Jianwei
Beijing 100085 (CN)**
• **WANG, Yang
Beijing 100085 (CN)**
• **MA, Rong
Beijing 100085 (CN)**
• **SUN, Yiping
Beijing 100085 (CN)**
• **TIAN, Xinsheng
Beijing 100085 (CN)**
• **ZHANG, Jun
Beijing 100085 (CN)**
• **LI, Ziqiang
Beijing 100085 (CN)**

(74) Representative: **Dolphin, Kirsty Mairi et al
Venner Shipley LLP
200 Aldersgate
London EC1A 4HD (GB)**

Remarks:
The complete document including Reference
Table(s) and the Sequence Listing(s) can be
downloaded from the EPO website

(54) **FUSION PROTEIN OF INTERLEUKIN-2 AND APPLICATION THEREOF IN ALS**

(57) Provided are a fusion protein of interleukin 2 and application thereof in the treatment or prevention of amyotrophic lateral sclerosis (ALS).

EP 4 394 042 A1

**Description**

TECHNICAL FIELD

The present disclosure belongs to the technical field of biopharmaceutical. In particular, the present disclosure relates to a fusion protein of interleukin-2 and its use in the treatment or prevention of amyotrophic lateral sclerosis (ALS).

BACKGROUND

Amyotrophic lateral sclerosis is also called ALS, Lou Gehrig's disease, and motor neuron disease. For ALS patients, there is damage, degradation and death of the upper and lower motor neurons, progressive weakness and dystrophy of the limb, trunk, and thoracico-abdominal muscles, and even complete loss in the ability of the brain of controlling voluntary movements. ALS may be genetic, or be caused by other factors such as autoimmune mechanism, neurotrophic factor disorder, oxidative stress, mitochondrial dysfunction, excitotoxity and protein misfolding. People with ALS usually just live a few years, and have high mortality rate.

At present, ALS is mainly treated with drugs. Although the drugs can delay the progression of ALS, they cannot reverse the condition. There is no radical cure of ALS. Riluzole, a glutamate antagonist, has been approved by US FDA for the treatment of ALS with twice daily (50 mg per 12 hours). It can extend life by about 4 months. However, 10% of patients have liver injury, and cannot repair the existing damages in motor neurons. Edaravone, a brain protectant for scavenging free radicals, can effectively prevent the early progression of ALS and delay the deterioration of lung function. But it cannot reduce the mortality rate of the disease, nor does it delay the decline in neurological function. The edaravone is administrated twice a day, each of which is 30mg diluted in an appropriate amount of saline and is injected intravenously within 30 minutes. Thus, frequent intravenous infusion is required during the therapeutic process, which is labor-intensive, costly and time-consuming, and has poor compliance of patients.

There is an urgent need for a drug for treating or preventing amyotrophic lateral sclerosis with a long administration interval.

SUMMARY OF THE INVENTION

The purpose of the disclosure is to provide a fusion protein of interleukin 2 and its use in the treatment or prevention of amyotrophic lateral sclerosis (ALS), with prolonged administration interval, alleviated pain of patients, reduced cost, and improved compliance and life quality of patients.

According to the first aspect of the present disclosure, a fusion protein of interleukin-2 is provided. The fusion protein comprises a human interleukin 2 or its variant, and a human serum albumin or its variant.

The human interleukin 2 or its variants may include: an amino acid sequence as shown in SEQ ID NO:1; or, an amino acid sequence having at least 90% sequence identity with the amino acid sequence shown in SEQ ID NO: 1.

The human serum albumin or its variants may include: an amino acid sequence as shown in SEQ ID NO:2; or, an amino acid sequence having at least 90% sequence identity with the amino acid sequence as shown in SEQ ID NO:2.

In the present disclosure, the amino acid sequence as shown in SEQ ID NO: 1 is human interleukin 2 with 133 amino acid residues, and the amino acid sequence as shown in SEQ ID NO:2 is human serum albumin with 585 amino acid residues.

In some embodiments, the fusion protein of the interleukin 2 may include the amino acid sequence as shown in SEQ ID NO: 1 and the amino acid sequence as shown in SEQ ID NO:2.

In some embodiments, the amino acid at position 125 of the amino acid sequence as shown in the SEQ ID NO: 1 is not cysteine. The human interleukin 2 (IL-2) has an intrachain disulfide bond. When the amino acid at position 125 of the mature protein is cysteine, it may tend to form mismatched disulfide bonds with the other two cysteines, resulting in the loss of the activity of IL-2.

In some embodiments, the amino acid at position 125 of the amino acid sequence as shown in SEQ ID NO: 1 may be replaced with serine or alanine. The mismatched disulfide bonds can be avoided through the mutation of the amino acid residue at position 125 of human interleukin 2 to serine or alanine, allowing the human interleukin 2 to remain active.

In some embodiments, the human interleukin 2 or its variant may be linked to the human serum albumin or its variant directly or via a linking peptide.

In some embodiments, the linking peptide is provided between the human interleukin 2 or its variant and the human serum albumin or its variant. There may be a large interval between the two units contained in the fusion protein of interleukin 2, so that the human interleukin 2 has the maximum probability of binding to the interleukin 2 receptor.

In some embodiments, the linking peptide may have a general formula of $(G_nS)_m$, in which n or m is an integer selected from 1 to 10.

In some embodiments, the linking peptide may have a general formula of $(G_nS)_m$, in which n is an integer selected from 1 to 4 and m is an integer selected from 0 to 3.

In some embodiments, the linking peptide may have a general formula of $(G_nS)_m$, in which n is an integer selected from 1-4 and m is an integer selected from 1-3.

In some embodiments, the linking peptide may have an amino acid sequence of GGGGSGGGGS.

In some embodiments, the linking peptide may have an amino acid sequence of GGGGS.

In some embodiments, the fusion protein may have a signal peptide at the N-terminal thereof. The addition of the signal peptide to the N-terminal of the fusion protein can further increase the expression level of the fusion protein.

In some embodiments, the signal peptide may be derived from the signal peptide of CD33 for secretion.

In some embodiments, the nucleic acid sequence encoding the signal peptide may be shown as SEQ ID NO:5.

In some embodiments, the fusion protein may have an amino acid sequence as shown in SEQ ID NO: 4.

In some embodiments, the fusion proteins may comprise: from N-terminal, human interleukin 2-linking peptide-human serum albumin, or human serum albumin -linking peptide-human interleukin 2.

In some embodiments, the amino acid sequence of SEQ ID NO:4 may be a protein having 728 amino acid residues, of which amino acids 1 to 133 are the human interleukin 2, amino acids 134 to 143 are the linking peptide GGGGSGGGGS, and amino acids 144 to 728 are the human serum albumin.

According to the second aspect of the present disclosure, an isolated nucleic acid molecule is provided. The nucleic acid molecule may encode the fusion protein provided in the first aspect of the present disclosure.

In some embodiments, the fusion protein may be encoded by the nucleotide sequence as shown in SEQ ID NO:3.

In the present disclosure, the codon optimization may be performed on the nucleotide sequences of the human interleukin 2 and human serum albumin disclosed on Genbank, according to the mammalian codon preference. The optimized nucleotide sequence of the fusion protein may be inserted in an expression vector, which is then transfected into a host cell for expression. The target protein may be purified, i.e., the fusion protein of interleukin 2.

The expression system of the fusion protein having the interleukin-2 and human serum albumin, can be transfected (for example, electrotransfer) into CHO cells with a plasmid carrying the fusion gene encoding the interleukin-2 and human serum albumin. Unexpectedly, a CHO monoclonal cell line which can stably and efficiently express the human recombinant protein, is obtained. Further, the monoclonal cell line of the present disclosure can express and secrete the fusion protein having the interleukin 2 and human serum albumin (i.e., the fusion protein of interleukin 2). It can greatly prolong the plasma half-life of human interleukin 2, and thus can be used for preparing the midicament associated with the expression of human interleukin 2.

In some embodiments, the expression vector may be selected from pEE14.4, pcDNA3.1 and pEE6.4, and preferably be pEE14.4.

According to the third aspect of the present disclosure, an expression system is provided. The expression system may comprise a CHO cell. The CHO cell may contain the nucleic acid molecule provided in the second aspect of the present disclosure.

The expression system may express the fusion protein provided in the first aspect of the present disclosure.

The present disclosure unexpectedly finds that the CHO cell, especially the CHO-K1 cell line, has a high expression level of the fusion protein, up to 4g /L. The expressed fusion protein of interleukin 2 is highly active, and can strongly promote, *in vitro,* the proliferation of specific cells such as NK, T and/or $T_{reg}$ cells.

In some embodiments, the expression system may be a CHO-K1 cell, which is deposited in China General Microbiological Culture Collection Center on May 26, 2022, under CGMCC No. 45173.

According to the fourth aspect of the present disclosure, a pharmaceutical composition is provided. The pharmaceutical composition may comprise a fusion protein provided in the first aspect of the present disclosure, and a pharmaceutically acceptable carrier.

In some embodiments, the pharmaceutical composition may be in a dosage form of injection, tablet, or capsule.

In some embodiments, the pharmaceutical composition may be in a dosage form of solution for injection or lyophilized powder for injection.

In some embodiments, the pharmaceutical composition may include a pharmaceutically acceptable carrier.

In some embodiments, the carrier may be selected from excipient, diluent, filler, binder, wetting agent, disintegrant, absorption enhancer, surfactant, adsorption support, and/or stabilizer.

According to the fifth aspect of the present disclosure, provided is a method for treating or preventing amyotrophic lateral sclerosis (ALS). The method may comprise administrating to a subject of an effective amount of the fusion protein provided by the first aspect of the present disclosure, or the pharmaceutical composition provided by the fourth aspect of the present disclosure.

In some embodiments, the fusion protein or the pharmaceutical composition may be administered by injection.

In some embodiments, the fusion protein or the pharmaceutical composition may be administered by subcutaneous or intravenous infusion.

In some embodiments, the fusion protein or the pharmaceutical composition may be administered by subcutaneous injection.

In some embodiments, the fusion protein may be administered at $3\times10^4$IU to $1\times10^6$IU each dose.

In some embodiments, the pharmaceutical composition may be administered at $3\times10^4$IU to $1\times10^6$IU each dose, as measured by the fusion protein therein.

In some embodiments, the fusion protein may be administered every 7-28 days.

In some embodiments, the fusion protein may be administered every 14-28 days.

In some embodiments, the pharmaceutical composition may be administered every 7-28 days.

In some embodiments, the pharmaceutical composition may be administered every 14-28 days.

According to the sixth aspect of the present disclosure, provided is use of the fusion protein provided in the first aspect of the present disclosure in the treatment or prevention of amyotrophic lateral sclerosis (ALS).

In some embodiments, the fusion protein may be used for the prevention or treatment of the decrease in limb strength, body weight, and/or neurological function caused by ALS.

According to the seventh aspect of the present disclosure, provided is use of the pharmaceutical composition provided in the fourth aspect of the present disclosure in the treatment or prevention of amyotrophic lateral sclerosis (ALS).

In some embodiments, the pharmaceutical composition may be used for the prevention or treatment of the decrease in limb strength, body weight, and/or neurological function caused by ALS.

According to the eighth aspect of the present disclosure, provided is use of the fusion protein provided in the first aspect of the present disclosure in the preparation of a medicament for treating or preventing amyotrophic lateral sclerosis (ALS).

In some embodiments, provided is use of the fusion protein in the preparation of a medicament for preventing or treating the decrease in limb strength, body weight, and/or neurological function caused by ALS.

According to the ninth aspect of the present disclosure, provided is use of the pharmaceutical composition provided in the fourth aspect of the present disclosure in the preparation of a medicament for treating or preventing amyotrophic lateral sclerosis (ALS).

In some embodiments, provided is use of the pharmaceutical composition in the preparation of a medicament for preventing or treating the decrease in limb strength, body weight, and/or neurological function caused by ALS.

The fusion protein of interleukin-2 of the present disclosure has high biological activity and long plasma half-life.

The fusion protein of interleukin 2 of the present disclosure is capable of treating or preventing amyotrophic lateral sclerosis.

The fusion protein of interleukin 2 of the present disclosure is capable of increasing limb strength in ALS patients.

The fusion protein of interleukin 2 of the present disclosure can slow down the loss in body weight of ALS patients.

The fusion protein of interleukin 2 of the present disclosure can improve the neurological function of ALS patients.

The fusion protein of interleukin 2 of the present disclosure can improve the survival rate and prolong the survival cycle of ALS patients.

The fusion protein of interleukin 2 of the present disclosure has an excellent therapeutic effect, e.g. in improving the survival rate, survival cycle, motor nerve function, and weight loss of ALS patients.

Moreover, the fusion protein of interleukin 2 provided by the present disclosure has slowed-down hydrolysis in patient, significantly prolonged interval time of the administration, enhanced therapeutic effect, reduced total administration dosage. Correspondingly, the fusion protein of the present disclosure can relieve the patient's pain, reduce treatment cost, reduce the occurrence of adverse reactions, thereby improving the compliance and life quality of the patients. Therefore, the present disclosure brings hope to the recovery of ALS patients.

DESCRIPTION OF THE DRAWINGS

FIG. 1 shows the reducing electrophoresis results of the supernatants of IL-2-HSA / CHO-K1 clones cultured in a well plate. 20 μL of the supernatants were loaded for each lane.

FIG. 2 shows the non-reducing electrophoresis results of the fed-batch culture supernatants on D13 of IL-2-HSA/CHO-K1 clones which were screened, and the protein expression of each cell line. 5 μL of the supernatants were loaded for each lane. FIG. 2A shows the non-reducing electrophoresis results of the fed-batch culture supernatants of the screened IL-2-HSA / CHO-K1 clones on D13; FIG. 2B shows the protein expression of each cell line.

FIG. 3 shows the non-reducing electrophoresis results of the fed-batch culture supernatants on D13 of IL-2-HSA / CHO-K1 clone # 9 which was subjected for monoclonal screening, and the protein expression of each cell line. 3 μL of the supernatants were loaded for each lane. FIG. 3A shows the non-reducing electrophoresis results of the fed-batch culture supernatant on D13 of IL-2-HSA / CHO-K1 clone # 9 which was subjected for monoclonal screening;

FIG. 3B shows the protein expression of each cell line.

FIG. 4 shows the non-reducing electrophoresis results of the fed-batch culture supernatants on D13 of IL-2-HSA / CHO-K1 clone # 9-6 which was subjected for monoclonal screening, and the protein expression of each cell line. 2 μL of the supernatants were loaded for each lane. FIG. 4A shows the non-reducing electrophoresis results of the fed-batch culture supernatants on D13 of IL-2-HSA / CHO-K1 clone # 9-6 which was subjected for monoclonal screening; FIG. 4B shows protein expression of each cell line.

FIG. 5 shows the kinetics curve of the cultured IL-2-HSA/CHO-K1 cells. The cells were fed-batch cultured in 125 mL shaker with an initial cell density of $0.3 \times 10^6$ cells / mL, an initial culture volume of 25 mL and a maximum viable cell density of up to $19.3 \times 10^6$ cells / mL.

FIG. 6 shows the dynamic curve of the protein expression of the supernatants on D7-D13 of the IL-2-HSA / CHO-K1 cells which were seeded at low-density and cultured.

FIG. 7 shows the non-reducing electrophoresis results of the supernatants of the IL-2-HSA / CHO-K1 cells. 1 μL of the supernatants were loaded for each lane.

FIG. 8 shows the kinetics curve of the cultured IL-2-HSA/CHO-K1 cells. The cells were fed-batch cultured in a 1L shaker with an initial cell density of $2 \times 10^6$ cells / mL, an initial culture volume of 300 mL and a maximum viable cell density of up to $16 \times 10^6$ cells / mL.

FIG. 9 shows the dynamic curve of the protein expression of the supernatants on D0-D10 of the IL-2-HSA / CHO-K1 cells which were seeded at high-density and cultured.

FIG. 10 shows the non-reducing electrophoresis results of the supernatants of the IL-2-HSA / CHO-K1 cells. 5 μL of the supernatants were loaded for each lane.

FIG. 11 shows the proliferation curve of NK-92 stimulated by IL-2-HSA.

FIG. 12 shows the proliferation curve of CTLL-2 stimulated by IL-2-HSA.

FIG. 13 shows the changes of the body weight in the B6SJL-Tg (SOD1-G93A) transgenic mouse models with spontaneous ALS.

FIG. 14 shows the grip time of the B6SJL-Tg (SOD1-G93A) transgenic mouse models with spontaneous ALS tested in the cage.

FIG. 15 shows the holding time of the B6SJL-Tg (SOD 1-G93A) transgenic mouse models with spontaneous ALS in the rotarod test.

FIG. 16 shows the neurological scores of the B6SJL-Tg (SOD 1-G93A) transgenic mouse models with spontaneous ALS.

FIG. 17 shows the survival rates of the B6SJL-Tg (SOD 1-G93A) transgenic mouse models with spontaneous ALS.

FIG. 18 shows the survival cycles of the B6SJL-Tg (SOD 1-G93A) transgenic mouse models with spontaneous ALS .

FIG. 19 shows the binding abilities of the IL-2-HSA fusion protein to IL-2R from different species (i.e., human, dog, rat, mouse).

FIG. 20 shows the *in vitro* proliferation effects of the IL-2-HSA fusion protein and rhIL-2 on $T_{reg}$ (CD3$^+$CD4$^+$CD25$^+$CD127$^{low/-}$) subpopulations in human PBMC.

FIG. 21 shows the flow cytometry results of $T_{reg}$ (CD3$^+$CD4$^+$CD25$^+$CD127$^{low/-}$) subpopulations in human PBMC.

**DESCRIPTION OF PARTICULAR EMBODIMENTS OF THE INVENTION**

The invention is further illustrated through the examples below. It should be understood that the examples of the present invention are only intended to illustrate the present invention but not to limit it. Any simple improvement made to the present invention under the concept of the present invention is within the scope of protection claimed by the present invention.

Definition

The term "fusion protein" as used herein means a bioactive polypeptide (usually TCR or an antibody) and an effector molecule (usually a protein or peptide sequence) which are covalently linked by any appropriate method such as recombinant, chemical or other methods. If desired, one or more molecules may be fused via a linking peptide. Alternatively, the linking peptide may be used to assist in the construction of the fusion molecule. The fusion molecule is particularly preferred to be a fusion protein. In general, the fusion molecules may also include the conjugated molecules.

The terms "expression vector" and "expression construct" as used herein can be used interchangeably. When the above isolated nucleic acid molecules are connected to the vector, the nucleic acid sequence can be directly or indirectly connected to the regulatory elements on the vector, as long as these regulatory elements can regulate the translation and expression of the nucleic acid molecules. These regulatory elements can derived directly from the vector itself, or can be exogenous, i.e., be not derived from the vector itself. That is, the nucleic acid molecule is operationally connected to the regulatory element(s). In this disclosure, "operationally connected" means that a foreign gene is connected to the vector so that the regulatory element(s) in the vector (e.g. transcription- and translation- regulatory sequences) can play the intended functions of regulating the transcription and translation of the foreign gene. Of course, the polynucleotides used for encoding the heavy and light chains of an antibody can be inserted independently into different vectors, often into the same vector. Common vectors may include, for example, plasmids, bacteriophages, and the like.

Compared to a sequence, a variant having "at least 90% sequence identity" with the sequence as used herein may include an amino acid sequence having at least 90%, at least 91%, at least 92%, at least 92%, at least 93%, at least 94%, at least 95%, at least 96%, at least 97%, at least 98%, at least 99% or 100% sequence identity with the sequence, and having identical or similar fuction to that of the sequence.

The term "pharmaceutically acceptable carrier" as used herein may include any physiologically compatible solvent, dispersion medium, coating, antibacterial agent, antifungal agent, isotonic agent, delayed absorber, and the like. Particular examples can include one or more selected from, e.g. water, saline water, phosphate buffered saline (PBS), glucose, glycerol, ethanol, and their combinations. In many cases, the pharmaceutically acceptable carrier may include isotonic agents, such as sugars, polyols (e.g., mannitol, sorbitol), or sodium chloride. The pharmaceutically acceptable carrier may also include a trace amount of auxiliary substances, such as wetting agents, emulsifiers, preservatives or buffers, to extend the shelf life or potency of the protein.

The term "subject" as used herein refers to subjects who have ALS or are at risk of developing ALS. Subjects can include animals, preferably be mammals, and more preferably be humans.

The term "treatment" as used herein includes playing a role in the specific disease, disorder or condition, reducing the severity of the disease, disorder or condition, or delaying or slowing the progression of the disease, disorder or condition.

The term "prevention" as used herein means the likelihood of at least reducing the risk (or susceptibility) to developing a disease or condition (i.e., stoping at least one clinical symptom of the disease that has not developed in a patient who may be exposed or susceptible to the disease but has not experienced or showed the symptoms of the disease).

The term "effective quantity" as used herein means an amount of the therapeutic, prophylactic and/or diagnostic agent which is sufficient for the treatment, relievement, improvement, reduction of the symptoms, prevention, suppression of the progression, reduction of the severity, and/or reduction of the incidence of a disease, condition, and / or symptom when administered to a subject having or being susceptible to the disease, condition, and / or symptom.

The term "dosing" or "dosage" as used herein means an amount that can relieve or delay the progression of a disease, or degenerative or traumatic conditions. The dosage can be determined depending on the specific disease to be treated and other factors, including age, weight, health status, severity of the disease, administration route, frequency of treatment, and whether other medications are accompanied during treatment.

The term "amyotrophic lateral sclerosis" or "ALS" as used herein refers to a kind of motor neuron disease, involving the upper motor neurons (e.g., brain, brain stem, spinal cord), and the lower motor neurons (e.g. cranial nerve nucleus, spinal cord anterior horn cells) and their innervated trunk, limbs and head-facial muscles. ALS is clinically manifested as mixed paralysis with injuries of the upper and lower motor neurons. ALS is substantially classified into familial- and sporadic ALS.

The solutions of the present invention will be illustrated in combination with the examples below. Those skilled in the art should understand that the following examples are used only to illustrate the invention and not be deemed as limiting the scope of the invention. In the following, the description of the commonly known techniques is omitted to avoid unnecessarily confusing the concept of this disclosure. Such techniques are described in many publications, such as Molecular Cloning: A Laboratory manual (Fourth Edition) (Cold Spring Harbor Laboratory Press).

The examples, which are not indicated with the specific technology or conditions, are performed through the technology or conditions described in the literatures in the field or according to the product specifications. The reagents or instruments used, which are not indicated with the manufacturers, are conventional products that can be available on the market.

The inventors found the decrease of the regulatory T ($T_{reg}$) cells in ALS patients is associated with the increased severity of the disease, and can predict the progression of the disease and the survival rate. It suggests that they may be a potential therapeutic target. In addition, the production, activation, and survival of the $T_{reg}$ cells are completely dependent on cytokine interleukin 2 (IL-2). Based on this, the inventors carried out the following experiments.

Illustrations of the terms and expressions involved in the examples are given below.

IL-2-HSA fusion protein is an abbreviated form of the fusion protein of interleukin 2.

IL-2 refers to human interleukin 2.

HSA refers to human serum albumin.

$^{125}$Ala IL-2 refers to a mutant of interleukin-$_2$ having an amino acid sequence in which the amino acid at position 125 is alanine. See the amino acid sequence shown by SEQ ID NO:1 for details.

The trade name of commercially available short-acting IL-2 is Xinjier and is manufactured by Beijing SL Pharm Co., LTD.

Edaravone is manufactured by Guorui Pharmaceutical Co., LTD, Sinopharm Group.

**Examples**

**Example 1: Construct a stably transfected cell line**

On the day before transfection, the density of CHO-K1 cells was adjusted to $0.5 \times 10^6$ cells/mL. On the day of transfection, high concentration endotoxin-free plasmids that had undergone linear treatment were prepared, the cell density and viability of CHO-K1 cells were measured, ensuring that the cell viability was greater than 97%. After the CHO-K1 cells were washed twice with CD CHO medium, an electroporation transfection reaction system was prepared: 700 $\mu$L of cell suspension and 40 $\mu$g of plasmid were mixed well and transferred into a 4 mm of electrode cup. The electrode cup was placed into an electroporator and the shock parameters were set to 300 V, 1000 $\mu$F. After one electric shock, the shocked cell suspension was transfer into preheated fresh CD CHO medium, and incubated at 37 °C for 20 minutes. The incubated cell suspension was inoculated evenly into a 96-well plate. 24 hours after the transfection, the cells were pressurized, added with CD CHO medium containing methionine sulfoximine (MSX) with the final concentration of 25-50 $\mu$M, and statically cultured under 5% $CO_2$, at 37 °C.

**Example 2: Screening of Monoclonal Cell Lines with High Expression**

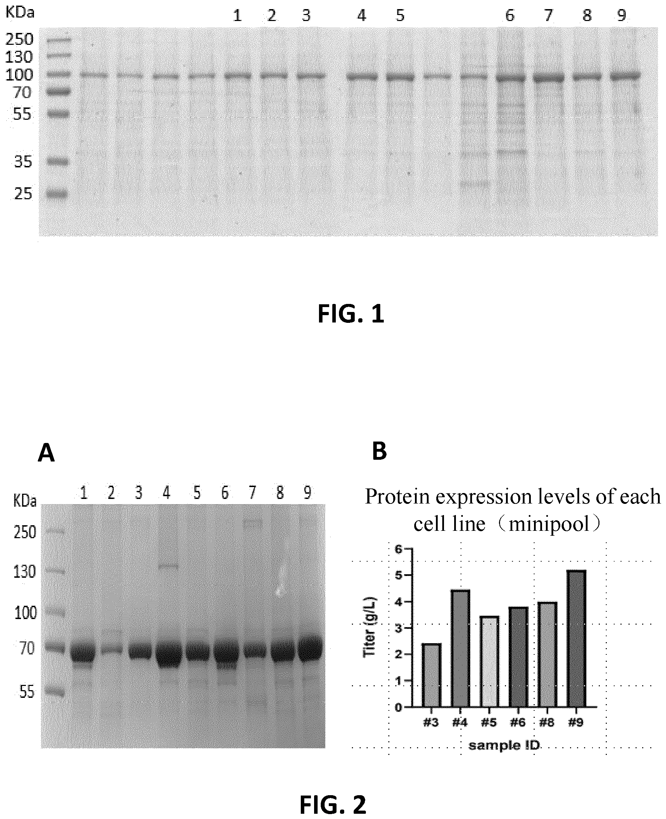

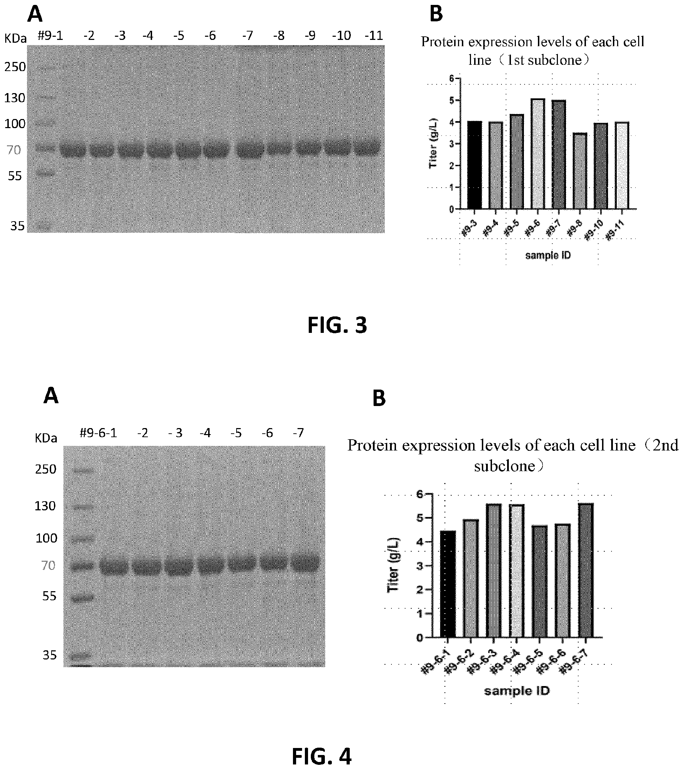

After growing to an appropriate size in the 96-well plate, the monoclonal cells were selected and transferred to a new 96-well plate. The selected monoclonal cells were incubated in 5% $CO_2$ at 37 °C for static culture. After the cells in the well grew to confluency, the supernatant was taken from the well plate for reducing electrophoresis to detect the expression of the fusion proteins. Nine clones with the highest expression levels were selected (see FIG. 1) and gradually expanded to shake flask culture. Nine clones were subjected to fed-batch culture in 25 mL shake flasks. The culture supernatant was harvested and identified by non-reducing electrophoresis (see FIG. 2A) and calculated for the protein expression level of each cell line (see FIG. 2B). The cell line # 9 with the highest expression level was selected. Cell line # 9 was screened for the monoclonal cell lines by using limited dilution approach. The cells were inoculated in 96-well plates with 0.3 cells/well to obtain 11 cell lines having high expression. These cell lines were then subjected to fed-batch culture in 25 mL shake flasks. The supernatants were identified by non-reducing electrophoresis (see FIG. 3A) and calculated for the protein expression level of each cell line (see FIG. 3B). The cell line #9-6 with the highest expression level was selected. The cell line #9-6 were further screened for monoclonal cell lines by using the limited dilution approach, resulting in 7 cell lines with high expression level. The obtained cell lines were subject to fed-batch culture in 25 mL shake flasks. The supernatants were identified by non-reducing electrophoresis (see FIG. 4A) and calculated for the protein expression level of each cell line (see FIG. 4B). The cell line #9-6-7 with high stability and expression level was selected as cell line IL-2-HSA/CHOK1 with stable and high expression of the protein.

**Example 3: Fed-Batch Culture of the Stable Cell Line in 125 mL Shake Flask**

On the day (recorded as D0) of starting the cultur for expression, IL-2-HSA/CHOK1 cells in 25 mL of basal medium containing 25-50 $\mu$M MSX were inoculated into a 125 mL of shake flask at a density of $0.3 \times 10^6$ cells/mL, and cultured with 5% $CO_2$ at 37 °C on a shaker at 135 rpm. On D4 of inoculation, the samples were taken and counted, and the culture temperature was decreased to 33 °C when the cell density reached to $10 \times 10^6$ cells/mL. On D5, fed-batch culture was carried out and the glucose concentration was controlled to 3 - 4 g/L. On D13 of culture, the culture was terminated, the cell culture supernatant was harvested. Then, the expression level of the fusion protein was measured to be 4.36 mg/mL.

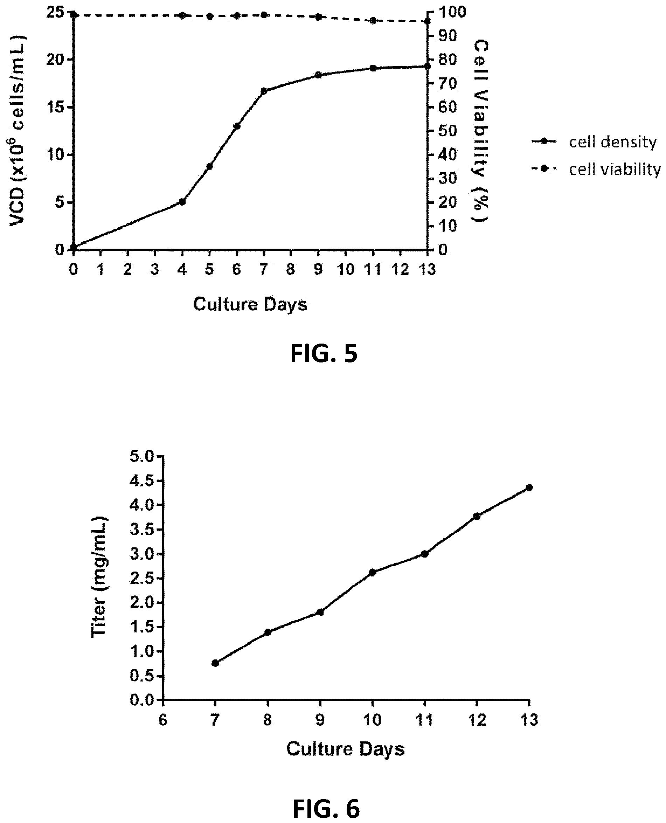

A culture kinetics curve of IL-2-HSA/CHOK1 cells is shown in FIG. 5. It can be seen from FIG. 5 that in the early stage of culture, the cells were in a logarithmic growth stage, with a rapid increase in density; in the later stage of culture, the cells entered a protein production stage, and the cell density tended to be stable. The maximum live cell density reached to $19.3 \times 10^6$ cells/mL.

A kinetics curve of the expression level of IL-2-HSA/CHOK1 cell supernatant is shown in FIG. 6. It can be seen from FIG. 6 that from D7 to D13, with the increase of culture days, the protein expression level of the cells showed a gradually increasing trend. The expression level on D13 was 4.36 mg/mL.

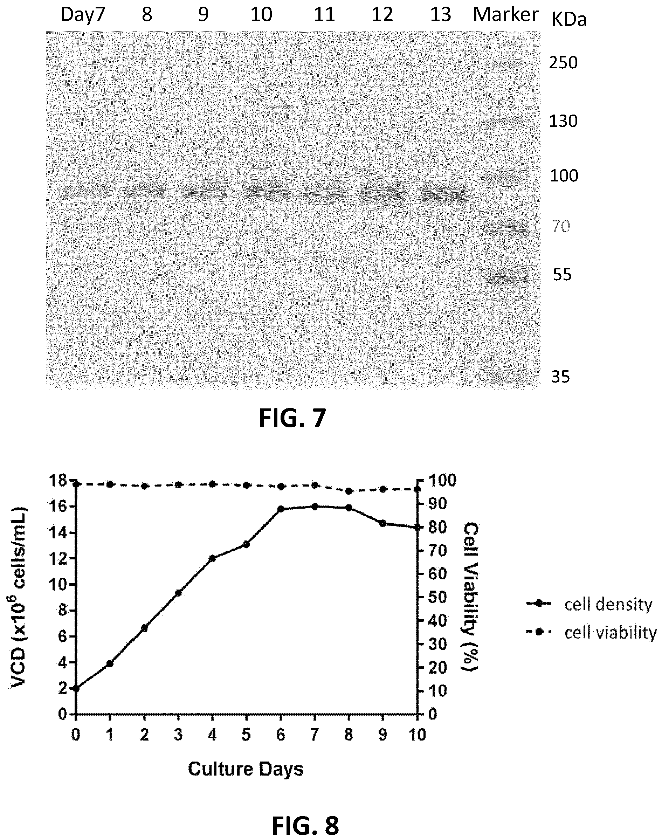

A non-reducing electrophoresis analysis of the protein expression of IL-2-HSA/CHOK1 cell supernatant is shown in FIG. 7. It can be seen from FIG. 7 that from D7 to D13, with the increase of culture days, the protein expression level of the cells showed a gradually increasing trend. The target protein had a single band without impurities.

**Example 4: Fed-Batch Culture of the Stable Cell Line in 1 L of Shake Flask**

IL-2-HSA/CHOK1 cells were inoculated into a 50 mL of shaking flask at a density of $2 \times 10^6$ cells/mL, and cultured with 5% $CO_2$ at 37 °C on a shaker at 135 rpm. The samples were taken and counted daily to observe cell status, and the basal medium containing 25-50 $\mu$M MSX was supplemented. The cell density was daily adjusted to $2 \times 10^6$ cells/mL until the volume of the cell culture solution reached to 300 mL. Then, stop supplement the basal medium and continue the culture, at which point it was recorded as D0. The samples were taken and counted daily and 1 mL of culture supernatant was retained. The culture temperature was decreased to 33 °C when the cell density reached to $6 \times 10^6$ - $7 \times 10^6$ cells/mL. On D2, the fed-batch culture was started and the glucose concentration was controlled to 3 g/L. On D10 of culture, the culture was terminated, the cell culture supernatants were harvested, and the protein expression levels of the supernatants were measured from D0 to D10. The expression level of fusion protein in the supernatant harvested on D10 was measured to be 3.12 mg/mL.

A kinetics curve of IL-2-HSA/CHOK1 cell culture is shown in FIG. 8. It can be seen from FIG. 8 that in the early stage of culture, the cells were in a logarithmic growth stage, with a rapid increase in density; in the later stage of culture, the cells entered a protein production stage, and the cell density tended to be stable. The maximum live cell density reached to $16 \times 10^6$ cells/mL.

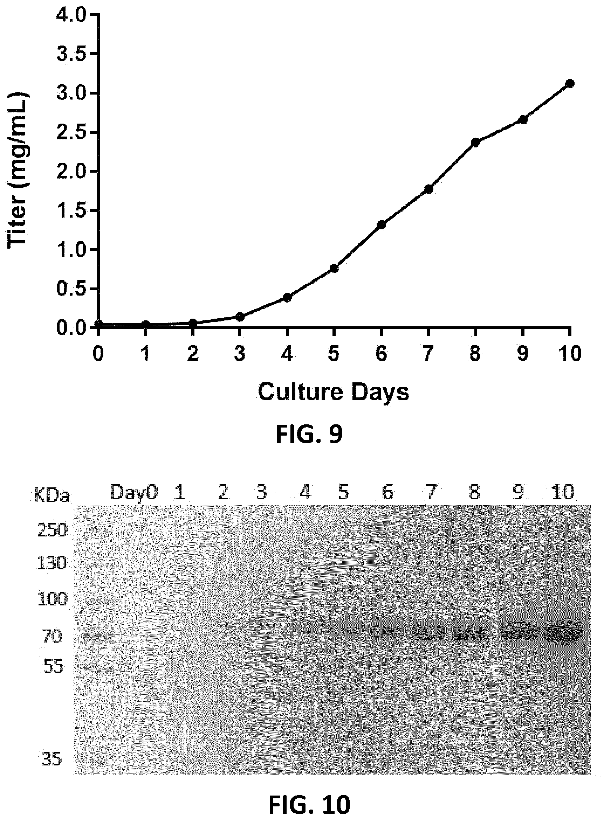

A kinetics curve of the expression level of IL-2-HSA/CHOK1 cell supernatant is shown in FIG. 9. It can be seen from FIG. 9 that with the increase of culture days, the protein expression level of the cell showed a gradually increasing trend.

A non-reducing electrophoresis analysis of the protein expression of IL-2-HSA/CHOK1 cell supernatant is shown in FIG. 10. It can be seen from FIG. 10 that with the increase of culture days, the protein expression level of the cells showed a gradually increasing trend. The target protein had a single band and no impurities.

Compared with the fed-batch culture in shake flasks in the prior art, the cell density and cell survival rate of the present disclosure are very high, as shown in FIG. 8. The cell density reaches to $14 \times 10^6$ - $16 \times 10^6$ cells/mL on D6 to D10. The protein expression level of the cells of the present disclosure is also very high, as shown in FIG. 9. The expression level on D10 is 3.12 mg/mL. In the present disclosure, even at the cell density of $14 \times 10^6$ - $16 \times 10^6$ cells/mL, the expression level is also high, indicating that the cells remain high activity at such a cell density.

**Example 5: Assay of Effect of IL-2-HAS on NK-92 Cell Proliferation**

In this example, NK-92 cells (ATCC® CRL-2407 ™, an IL-2 dependent NK cell line derived from the peripheral blood monocytes of a 50 year-old male patient with malignant non-Hodgkin's lymphoma) were utilized to evaluate the biological activity of IL-2-HSA.

(1) Cryopreserved NK-92 cells were taken out from a liquid nitrogen tank, thawed and cultured until reaching the logarithmic growth phase.

(2) Sufficient cells were collected by centrifugation, resuspended in the complete culture medium without IL-2, and cultured under starvation for 24 hours.

(3) The starved NK-92 cells were centrifuged, resuspended in the complete culture medium without IL-2, and counted. The cell density was adjusted to $5\times10^5$ cells/mL, and the cells were added into the 96-well plate at a volume of 90 $\mu$L per well.

(4) Preparation of sample solution: IL-2-HSA and rhIL-2 (R&D, Cat. No. 202-IL) samples were pre-diluted to 50.67 nM with the medium, and diluted to 9 concentrations at a 4-fold gradient. The diluted samples were added to corresponding wells of the 96-well plate at 10 $\mu$L/well, with three replicates for each concentration. For the negative control group, the medium was added at 10 $\mu$L/well correspondingly and mixed well.

(5) After culturing under 5% $CO_2$ at 37 °C for 72 hours, the melted and mixed MTS detection reagent was added to the above 96-well plate at 20 $\mu$L/well, shook well in an oscillator, and then incubated in a cell culture incubator at 37 °C, 5% $CO_2$ for additional 1 to 4 hours.

(6) After incubation, shook and mixed well. The absorbance was measured at a wavelength of 490 nm using a microplate reader.

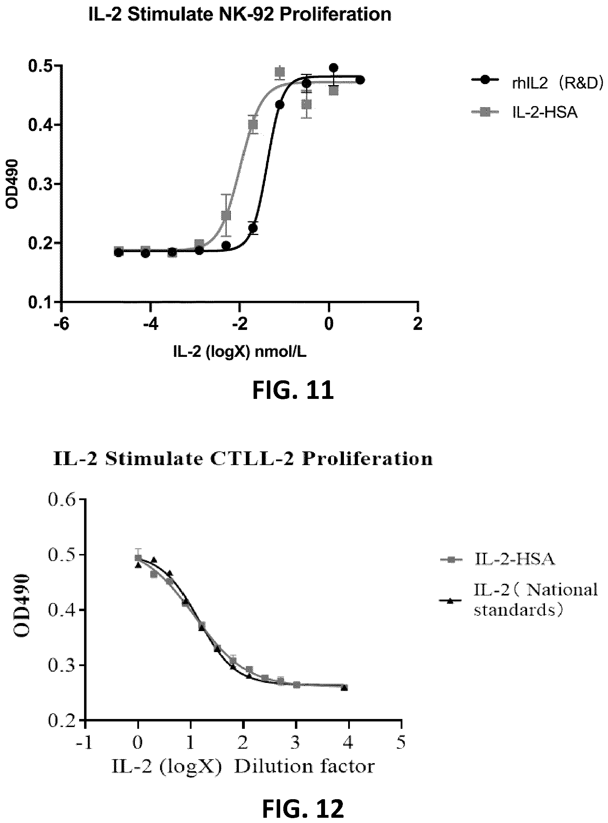

(7) The data was analyzed using GraphPad Prism 8 software, with the logarithm of drug concentration X as the horizontal coordinate and $OD_{490}$ as the vertical coordinate. The drug action curve was fitted with four parameters. The $EC_{50}$ values obtained are shown in Table 1, and the proliferation curve is shown in FIG. 11.

Table 1

|  | IL-2 (R&D) | IL-2-HSA |
|---|---|---|
| $EC_{50}$ (nM) | 0.0420 | 0.01047 |

It can be seen from FIG. 11 that IL-2-HSA induced NK-92 cell growth in a dose-dependent manner. Under the experimental condition, the activity of IL-2-HSA in stimulating NK-92 proliferation was higher than that of equimolar rhIL-2 (about 4 times).

**Example 6: Assay of Effect of IL-2-HAS on CTLL-2 Cell Proliferation**

In this example, CTLL-2 cells (ATCC® TIB™, a mouse cytotoxic T lymphocyte cell line, IL-2 dependent) were utilized to evaluate the biological activity of IL-2-HSA.

Referring to the human interleukin-2 biological activity assay (CTLL-2 cell/MTT colorimetric assay) in the Chinese Pharmacopoeia, CTLL-2 cells were inoculated into 96-well plate at a density of 30000 cells/well, and added with a series of gradient diluted national standards and IL-2-HSA, and cultured in 5% $CO_2$ at 37 °C for 18 to 24 hours. Then, MTS reagent was added and incubated for 1 to 4 hours. After shaking and mixing well, the absorbance was measured at a wavelength of 490 nm using a microplate reader. The data was analyzed using GraphPad Prism 8 software, with the logarithm of dilution X as the horizontal coordinate and $OD_{490}$ as the vertical coordinate. The drug action curve was fitted with four parameters. The $EC_{50}$ values obtained are shown in Table 2, and the proliferation curve is shown in FIG. 12.

The biological activity of IL-2-HSA is calculated using the following formula:

$$\begin{aligned} \text{biological activity of test sample IU/mL} \\ = \text{biological activity of standard IU/mL} \times \frac{\text{pre} - \text{dilution ratio of test sample}}{\text{pre} - \text{dilution ratio of standard}} \\ \times \frac{\text{dilution ratio of test sample equivalent to half effective amount of standard}}{\text{dilution ratio of semi} - \text{effective amount of standard}} \end{aligned}$$

After calculation, the specific activity of IL-2-HSA was $8.38\times10^6$ IU/mg, comparable to the specific activity (not less than $1\times10^7$ IU/mg) of IL-2 as specified in the pharmacopoeia. However, the difference between the molecular weights of IL-2-HAS and IL-2 was about 5 times. Thus, the specific activity of IL-2-HSA was higher.

Table 2

|  | IL-2 (National standard) | IL-2-HSA |
|---|---|---|
| $EC_{50}$(dilution ratio) | 13.90 | 11.68 |

**Example 7: Preventive and therapeutic effects of the IL-2-HSA fusion protein on spontaneous ALS disease of the mouse model**

This trial included 19 animals. G1 group: 4 wild-type C57BL / 6 mice, as the wild-type control group. 15 B6SJL-Tg (SOD1-G93A) mice were randomly divided into 3 groups by body weights, respectively: G2 group: model control group; G3 group: Edaravone group; G4 group: VDJ010 group, i.e. IL-2-HSA fusion protein group. Among them, G 2 group: 4 mice, G3 group: 5 mice; G4 group: 6 mice. The specific information of the animal grouping and dosing regimen is shown in Table 3.

Table 3

| Grouping | Number Of Animals | Drug name | Dosage (mg/kg) | Drug concentration (mg/ml) | Administration Route | Administration Frequency |
|---|---|---|---|---|---|---|
| G1 | 4 | no | N/A | N/A | N/A | N/A |
| G2 | 4 | 0.01M DPBS, without $Ca^{2+}$, $Mg^{2+}$ | 50 $\mu$L/ mouse | N/A | s.c. | Once a week for 10 days |
| G3 | 5 | Edaravone | 15mg/kg | 1.5mg/ml | i.p. | Once a day for 10 days |
| G4 | 6 | IL-2-HSA fusion protein | 100,000 IU/ 50 $\mu$L/ mouse | $10^6$ IU/bottle | s.c. | Once a week for 10 days |
| Note: 0.1ml/10g body weight, N/A: Not applicable; s.c.: subcutaneous injection; i.p.: intraperitoneal injection. | | | | | | |

Administration: ALS transgenic model of B6SJL-Tg (SOD 1-G93A) transgenic mice were treated on day 90 after birth; Edaravone was administrated once a day; and the IL-2-HSA fusion protein was administrated once a week.

**Testing index**

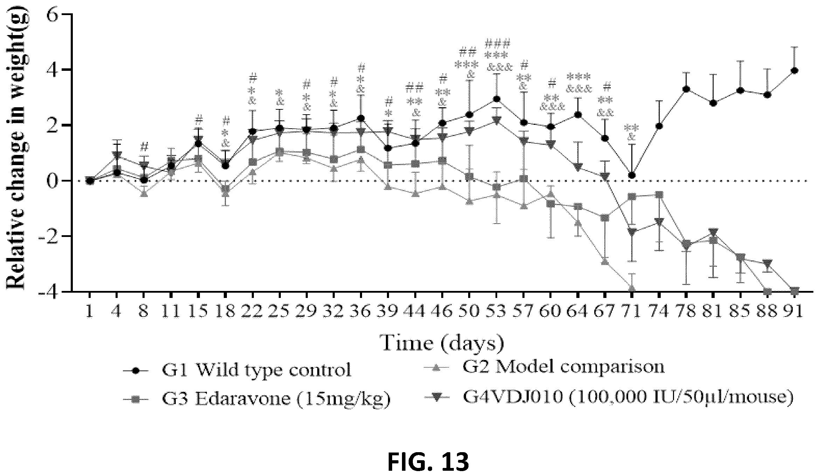

Weight: weighing in the morning, twice a week. Results are shown in FIG. 13. FIG. 13 shows the relative changes in the body weights of the mice (Dn-D1, g). For G2 group (model control group) vs. G1 group (wild type control group), *$P<0.05$, **$P<0.01$, ***$P<0.001$; for G3 group (edaravone group) vs. G1 group, &$P<0.05$, &&$P<0.01$, &&&$P <0.001$; for G4 group (VDJ010) vs. G1, @$P<0.05$, @@$P<0.01$, @@@$P<0.001$; for G3 group vs. G2 group, $P<0.05, $$P<0.01, $$$P<0.001; for G4 group vs. G2 group, #$P<0.05$, ##$P<0.01$, ###$P<0.001$. According to FIG. 13, the relatively changed body weights of the B6SJL-Tg (SOD1-G93A) transgenic mice decreased more compared with the wild type. It demonstrated that the ALS model was successfully established. The relatively changed body weights of the IL-2-HSA fusion protein group were higher than those of the edaravone and model control groups. Thus, the IL-2-HSA fusion protein could inhibit the weight loss of B6SJL-Tg (SOD 1-G93A) transgenic mice with better effect than edaravone.

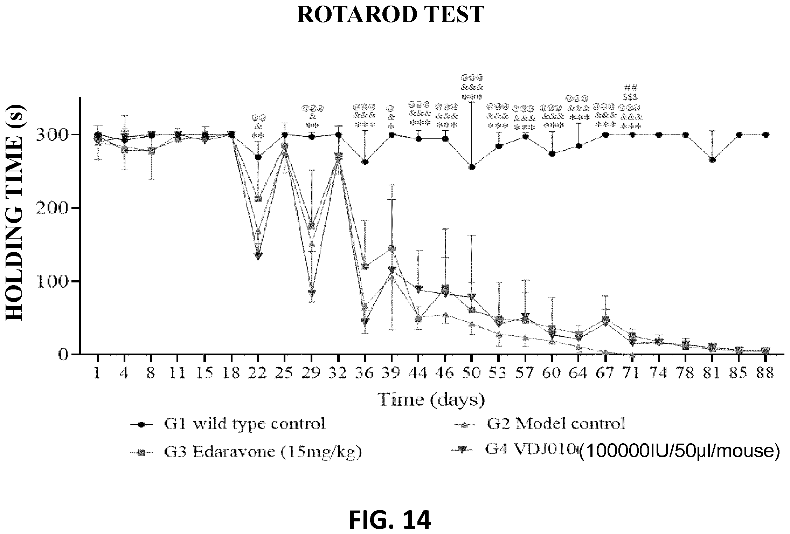

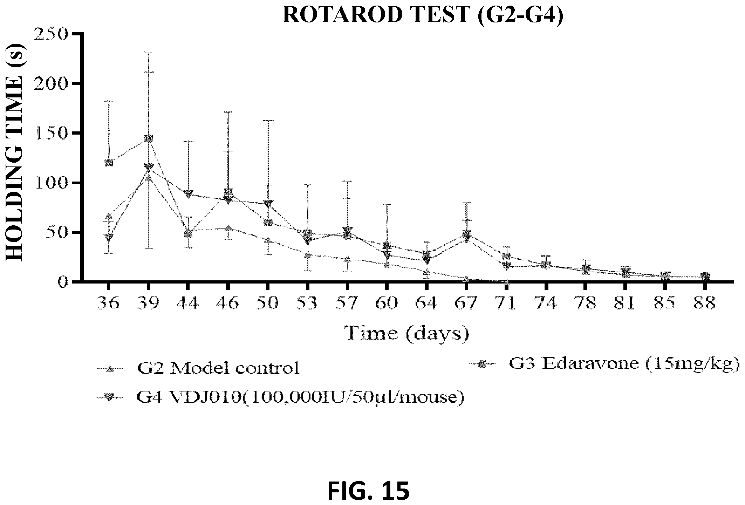

Rotarod test: the mice of the control group and the treatment group simultaneously started to rotate the rod twice a week from day 90. One week prior to recording experimental data, the mice were trained to adapt and learn the rotating of the rod, so that they could keep rotating the rod for 3 minutes (35 rpm/min). The rotarod instrument was operated to have acceleration of 35, slowly reach 35 rpm and maintained at this speed. Each mouse was measured three times for 300 seconds with an interval of 30 min. The three rotation times were averaged for statistics. The results are shown in FIGs 14 and 15, which show, for G2 group (model control group) vs. G1 group (wild-type control group), *$P<0.05$, **$P<0.01$, ***$P<0.001$, for G3 group (Edaravone group) vs. G1 group, &$P<0.05$, &&$P<0.01$, &&&$P <0.001$; for G4 group (VDJ010) vs. G1 group, @$P<0.05$, @@$P<0.01$, @@@$P <0.001$; for G3 group vs. G2 group, $P<0.05, $$P<0.01. $$$P<0.001; for G4 gorup vs. G2 group, #$P<0.05$, ##$P<0.01$, ###$P<0.001$. FIG. 14 shows the rotarod holding time, and FIG. 15 shows the rotarod holding time of G2 to G4 groups. According to FIGs. 14 and 15, the rotarod time of the mice in G2 group (model control group), G3 group (Edaravone group) and G4 group (VDJ010 group) were all shorter than that of G1 group (wild-type control group), demonstrating that the ALS model was successfully established. The rotarod time of the mice in G4 group (VDJ010 group) was comparable to that of G3 group (edaravone group), both are longer than the model control group. Thus, the IL-2-HSA fusion protein was able to improve the rotarod time and motor function of the ALS mice.

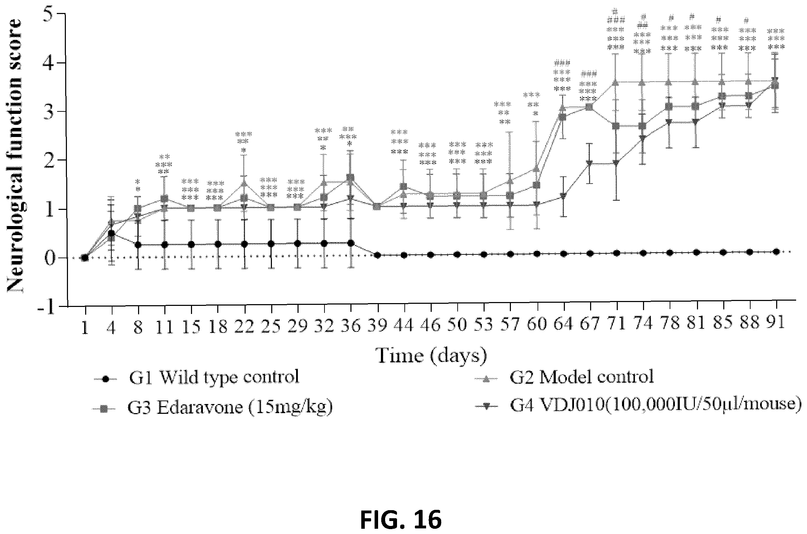

Neurological scoring: From day 90, the mice from G1 to G4 groups were scored for motor neurological function twice a week. This scoring criteria was based on the 0 to 4 evaluation system recommended by ALS therapy development institute (ALS TDI). For a score of 0: the hindlimbs of the mouse can fully splay during tail suspension, and can hold for 2 sec. For a score of 1: the hindlimbs of a mouse cannot fully splay or cannot splay during tail suspension, or tremble. For a score of 2: the toes of the mouse curl downwards at least twice at least twice during a 30 cm (12 inches), or any part of the foots is dragging along on the table. For a score of 3: there is severe paralysis or minimal joint movement, only the forelimbs are used for forward crawling. For a score of 4: the mouse can not turn over within 30 sec. The results are shown in FIG. 16, in which, for G2 group (model control) vs. G1 group (wild-type control), *P<0.05, **P<0.01, ***P <0.001; for G3 group (edaravone) vs. G1,&P<0.05, &&P<0.01, &&&P <0.001; for G4 group (VDJ010) vs. G1 group, @P<0.05, @@P<0.01, @@@P <0.001; for G3 group vs. G2 group, $P<0.05, $$P<0.01, $$$P <0.001; for G4 group vs. G2 group, #P<0.05, ##P<0.01, ###P<0.001. FIG. 16 shows the neurological scores. As shown in FIG. 16, the neurological scores of the mice in G2 group (model control group), G3 group (Edaravone group) and G4 group (VDJ010 group) were all higher than those in the wild-type control group, indicating that the nervous regulation on motion thereof was lower than that in G1 group (wild-type control group), and the transgenic mouse models (SOD1-G93A) with spontaneous ALS was successfully established. After day 44, the neurological scores of G4 group (VDJ010 group) were lower than those of G2 group (model control group) and G3 group (Edaravone group). The nervous regulation on motion of the mice in G4 group (VDJ010 group) was higher than that of G2 group (model control group) and G3 group (Edaravone group). It can be seen that the IL-2-HSA fusion protein can significantly improve the nervous function of transgenic mice (SOD1-G93A) with spontaneous ALS.

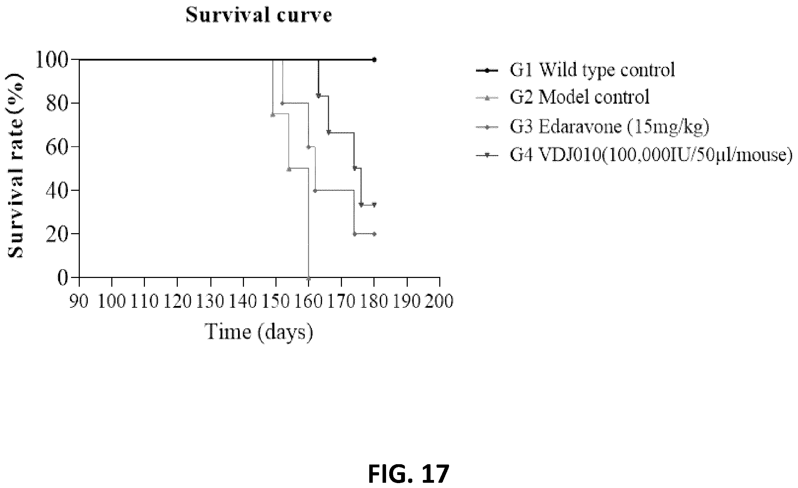

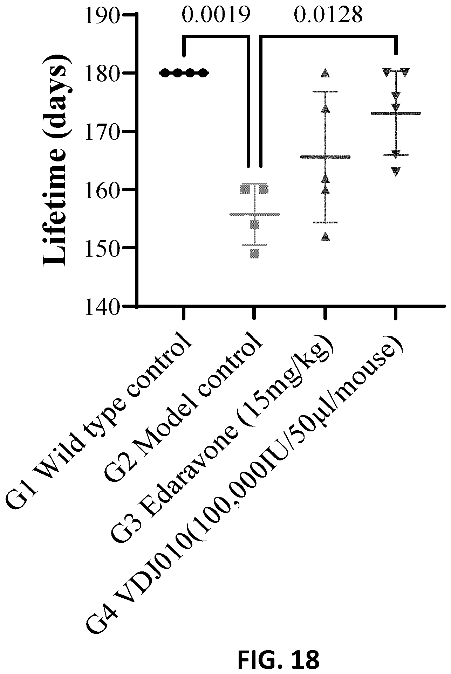

Observation of the survival period of the experimental animals: the experimental animals were observed for their physical state until the end of the expriments. The death time of each animal was recorded. The survival rates and the survival cycles of the mice were also calculated. The results are shown in FIGs. 17 and 18. FIG. 17 shows the survival curves of the experimental animals. FIG. 18 shows the survival cycles of the experimental animals. *P<0.05, **P<0.01, ***P<0.001. As can be seen from FIG.s 17 and 18, both the survival rates and survival cycles of the ALS mice in group G4 (group VDJ010) were higher than those in G2 group (model control group) and G3 group (Edaravone group). It can be seen that the IL-2-HSA fusion protein can significantly improve the survival rates and survival cycles of the transgenic mice (SOD1-G93A) with spontaneous ALS. With the changes of the body weights, neurological scores and survival cycles of mices, it shows that the IL-2-HSA fusion protein was superior to Edaravone in efficacy, and may produce less adverse reactions to the body with respect to Edaravone.

**Example 8: Affinity analysis of the IL-2-HSA fusion protein with IL-2Rs of different species**

The interaction between the IL-2-HSA fusion protein and IL-2 receptors (IL-2Rs) of different species (human, dog, rat, mouse) were analyzed by Bio-Layer Interferometry (BLI). IL-2Rs of different species were captured through ProA or hFc probes. The IL-2-HSA fusion protein was diluted to different concentration gradients. Then, the association and dissociation of the IL-2-HSA fusion protein with the IL-2Rs of different species attached to ProA or hFc probes were measured, to determine the affinity of the IL-2-HSA fusion protein with the IL-2Rs of different species. The specific processes are described as follows:

(1) Human IL-2R (hIL2R, BIOSYSTEMS, ILGH5257) and mouse IL-2R (mIL2R, BIOSYSTEMS, ILGM5253) were diluted to 30 nM and adsorbated to ProA probe at 400 rpm/min. Rat IL-2R (rIL2R, self-made, 20220111, gene accession number: IL2Rα_RAT P26897, IL2Rβ RAT P26896, IL2RγAAH79343.1) and dog IL-2R (CaIL2R, self-made, 20220421, gene accession number: IL2Rα O62802, IL-2RP F1PGA6, IL-2Ry_P40321) were diluted to 30 nM and adsorbated to hFc probe at 400 rpm /min.

(2) The IL-2-HSA fusion protein was diluted from 20 nM to 5 concentration gradients at a fold. A program was set so that the diluted IL-2-HSA fusion proteins was bound to the IL-2Rs attached to ProA or hFc probes, respectively, at fixed time. After the binding, the probes attached with the complex were then transferred to Q buffer without the analyte to dissociate the binding analyte.

(3) The probes were dipped into Regeneration buffer to remove residual binding analyte.

(4) The regenerated probes were placed in a 15% sucrose protective solution, and then stored at room temperature.

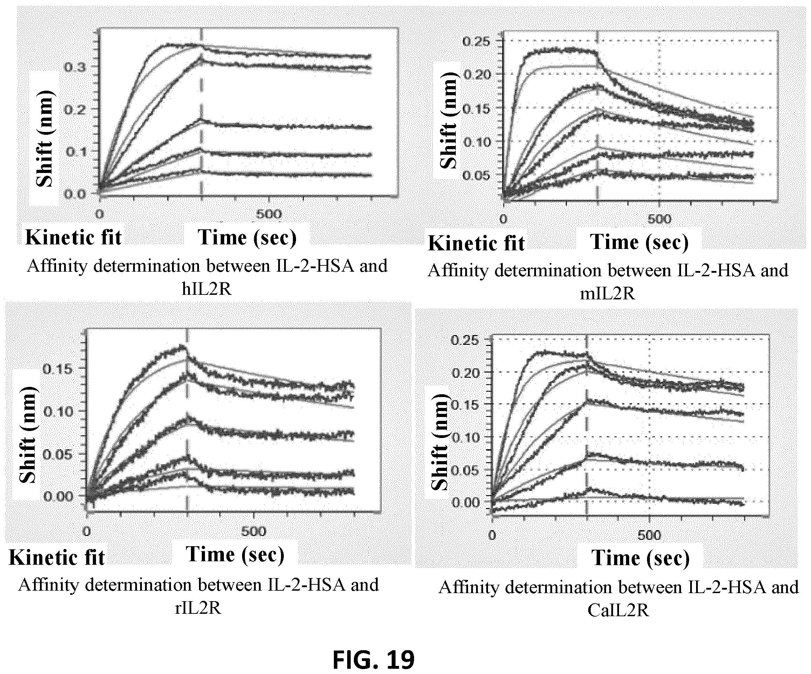

The binding abilities of the IL-2-HSA fusion protein with the IL-2Rs of different species are shown in FIG. 19 and Table 4.

Table 4

| Receptor | Sample Book | $k_{off}$(1/s) | $k_{on}$(1/Ms) | KD (M) |
|---|---|---|---|---|
| hIL2R | IL-2-HSA | 0.000169 | 1.96E+06 | 0.86E-10 |
| rIL2R | IL-2-HSA | 0.000549 | 1.77E+06 | 3.11E-10 |
| mIL2R | IL-2-HSA | 0.000887 | 1.65E+06 | 5.38E-10 |
| CaIL2R | IL-2-HSA | 0.000412 | 3.16E+06 | 1.30E-10 |

The above results indicate that: 1) the IL-2-HSA fusion protein binds to all the IL-2Rs of human, rat, mouse and dog, and has cross-species property; 2) the affinity of the IL-2-HSA fusion protein with the IL-2Rs of different species is ranked as follows: human> dog> rat> mouse.

**Example 9: Effect of the IL-2-HSA fusion protein on the *in vitro* proliferation of $T_{reg}$ cells from PBMC of healthy human**

In this example, the effect on the *in vitro* proliferation of $T_{reg}$ (CD3+CD4+CD25+CD127low/-) subpopulation from peripheral blood mononuclear cells (PBMCs) of healthy people of the IL-2-HSA fusion protein was compared with that of commercial rhIL-2 (R&D, Cat. No.: 202-IL) by using flow cytometry.

**1. Extraction of PBMC from human blood**

Fresh anticoagulant blood was collected from healthy people, and PBMCs were extracted with lymphocyte isolation solution (Ficoll-Paque PREMIUM, Cytiva, Cat. No.: 17-5442-02). 3-5 mL of lymphocyte isolation solution was added to a 15 mL centrifuge tube, the diluted blood sample (3 mL peripheral blood and 3 mL PBS mixed) was carefully added to the upper layer of the lymphocyte isolation solution, centrifuged at 400 g for 30-40 min. The layer of white blood cells were removed, washed twice with PBS, resuspended in RPMI-1640 medium + 10% FBS and counted.

**2. Drug treatment**

1) Cell density was adjusted to $2\times10^6$ cells/ mL with culture medium, inoculated in a 12-well plate at 1800 $\mu$L per well.

2) The IL-2-HSA fusion protein samples and rhIL-2 were prediluted to 10 $\mu$M with culture medium, diluted to six concentrations at a 10-fold gradient, and added to the corresponding wells of the 12-well plate at 200 $\mu$L/well. The culture medium was added to blank control wells accordingly at 200 $\mu$L/well.

3) Place in a cell culture incubator at 37°C, 5% $CO_2$, and grow continuously for 72 h.

**3. Flow-cytometric analysis**

1) Transfer the cell suspension to a new Ep tube, $2\times10^6$ cells/100 $\mu$L/tube.

2) Add PB450 anti-human CD3 (BD, Cat. No.: 558124,1:200), PC5.5 anti-human CD4 (BD, Cat. No.: 556924, 1:100), APC anti-human CD25 (BD, Cat. No.: 555434,1:50), PE anti-human CD127 (BD, Cat. No.: 557938, 1:100) respectively at a corresponding portion. Addtionally set negative control, monopositive control and isotype control. Incubate at 4°C for 30 min in dark place.

3) Centrifuge at 400 g for 5 min and wash twice with DPBS.

4) Resuspend with 300 $\mu$L DPBS, followed by testing on flow cytometer.

## 4. Test results

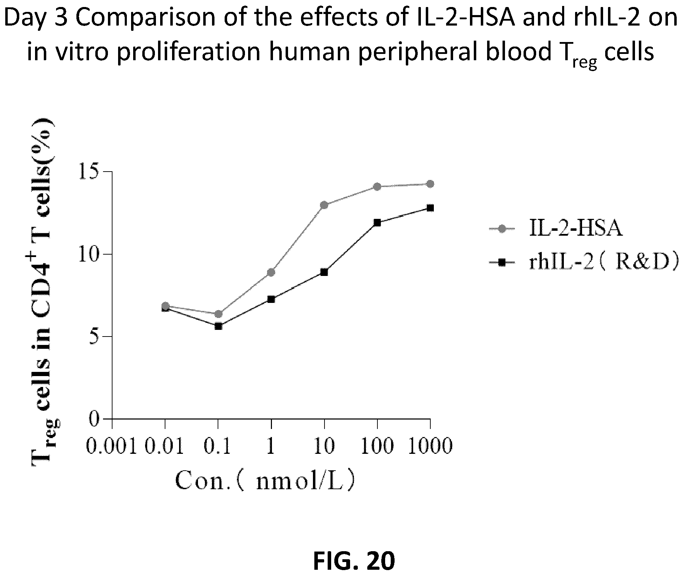

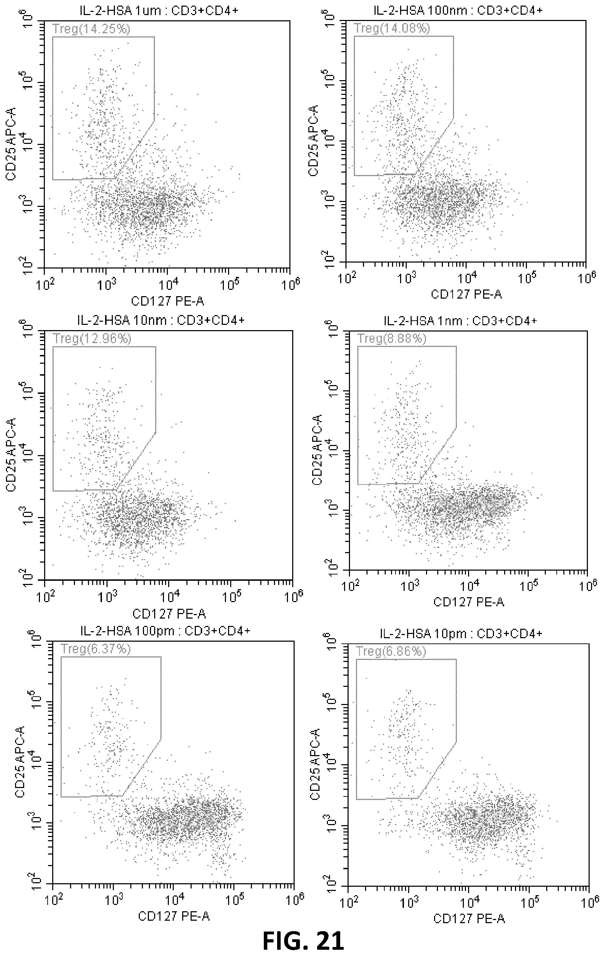

The effects of the IL-2-HSA fusion protein and rhIL-2 on the *in vitro* proliferation of $T_{reg}$ (CD3+CD4+CD25+CD127low/-) subpopulation in human PBMC are shown in FIGs. 20 and 21. FIG. 20 shows the comparison of the effects of the IL-2-HSA fusion protein and rhIL-2 on the proliferation of $T_{reg}$ cells in human PBMC on Day 3. FIG. 21 shows the flow-cytometric analysis results of $T_{reg}$ (CD3+CD4+CD25+CD127low/-) subpopulation in human PBMC.

According to the results as shown in FIG. 20, the proportion of $T_{reg}$ cells in human PBMC with increases in a dose-dependent manner after treating with the IL-2-HSA fusion protein for 72 h. In addition, the IL-2-HSA fusion protein has biological activity of promoting the proliferation of human $T_{reg}$ cell superior to rhIL-2.

**Example 10: Pharmacokinetics of the IL-2-HSA fusion protein in rat and dog with a single subcutaneous injection.**

In this example, the pharmacokinetics (PK) of the IL-2-HSA fusion protein was investigated in SD rats and Beagle dogs.

**(1) Pharmacokinetics of the IL-2-HSA fusion protein after a single subcutaneous and intravenous administration in SD rats.**

The trial included 40 SD rats, which had no administration history, and were randomly divided into 4 groups of 10 rats each, half male and half female. The animals in Groups 1-3 were administered with a single subcutaneous injection on the nape of neck. The low-, medium- and high-dosing groups were administered with $5 \times 10^5$ IU/kg, $1 \times 10^6$ IU/kg, $2 \times 10^6$ IU/kg, respectively. The animals in Group 4 were administered with tail intravenous injection at $1 \times 10^6$ IU / kg. All of the volumes of administration were 2 mL/kg. PK blood samples were collected from the test animals at the following time points:

Groups 1-3 (subcutaneous injection group) : before administration, and 1h, 2 h, 4 h, 8 h, 12 h, 24 h, 30 h, 36 h, 48 h, 72 h after administration;

Group 4 (intravenous injection group): before administration, and 2 min, 15 min, 1 h, 2 h, 4 h, 8 h, 12 h, 24 h, 48 h, 48 h, 72 h, 96 h after administration.

The concentrations of IL-2-HSA in the serum of SD rats were quantified by validated ELISA test. The pharmacokinetic parameters were calculated using noncompartmental analysis of WinNonlin software (Phoenix™, Version 8.1).

The major pharmacokinetic parameters are shown in Table 5, after the SD rats were administered with a single subcutaneous injection of different dosages ($5 \times 10^5$, $1 \times 10^6$ $2 \times 10^6$ IU/kg) of and a single intravenous injection of $1 \times 10^6$ IU/kg of the IL-2-HSA fusion protein.

**(2) Pharmacokinetics of the IL-2-HSA fusion protein after a single subcutaneous and intravenous injection in Beagle dogs.**

The trial included 24 Beagle dogs, which had no administration history, and were randomly divided into 4 groups of 6 dogs each, half male and half female. The animals in Groups 1-3 were administered with a single subcutaneous injection on the nape of neck. The low-, medium- and high-dosing group administered with $3 \times 10^5$ IU/kg, $6 \times 10^5$ IU/kg, $1.2 \times 10^6$ IU/kg, respectively. All of the volumes of administration were 0.6 mL/kg. The animals in Group 4 were administered with intravenous injection at $6 \times 10^5$ IU/kg. PK blood samples were collected from the test animals at the following time points:

Groups 1-3 (subcutaneous injection group): before administration, and 1h, 2 h, 4 h, 8 h, 12 h, 24 h, 36 h, 48 h, 60 h, 72 h, 96 h and 120 h after administration;

Group 4 (intravenous injection group): before administration, and 2 min, 15 min, 1h, 2 h, 4 h, 8 h, 12 h, 24 h, 48 h, 72 h, 96 h and 120 h after administration.

The concentrations of IL-2-HSA in the serum of Beagle dogs were quantified by validated ELISA test. The pharmacokinetic parameters were calculated using noncompartmental analysis of WinNonlin software (Phoenix™, Version 8.1).

The major pharmacokinetic parameters are shown in Table 6, after the Beagle dogs were administered with a single subcutaneous injection of different dosages ($3\times10^5$, $6\times10^5$, $1.2\times10^6$IU/kg) of and a single intravenous injection of $6\times10^5$ IU/kg of the IL-2-HSA fusion protein.

Table 6

| Species | SD rats | | | | | | | |
|---|---|---|---|---|---|---|---|---|
| Administration Route | Subcutaneous Injection | | | | | | Intravenous Injection | |
| Administration Dosage | $5\times10^5$IU/kg | | $1\times10^6$IU/kg | | $2\times10^6$IU/kg | | $1\times10^6$IU/kg | |
| Gender | female | male | female | male | female | male | female | male |
| PK parameters (mean, n =5) | | | | | | | | |
| $T_{1/2}$(h) | 3.21 | 4.21 | 3.72 | 3.78 | 4.52 | 3.82 | 5.24 | 5.40 |
| $T_{max}$(h) | 12.0 | 12.8 | 10.4 | 16.8 | 12.0 | 16.8 | - | - |
| $C_{max}$(n g/mL) | 180 | 145 | 410 | 269 | 939 | 556 | 5980 | 6362 |
| $AUC_{last}$(h*ng/mL) | 3376 | 2946 | 8593 | 5602 | 18787 | 13339 | 41544 | 47666 |
| Vd(mL/kg ) | 150 | 181 | 138 | 244 | 152 | 184 | 38.4 | 33.9 |
| CL (mL/h/kg ) | 31.9 | 30.6 | 25.4 | 42.0 | 23.6 | 32.1 | 5.12 | 4.40 |
| $MRT_{last}$(h) | 14.0 | 14.8 | 14.5 | 17.3 | 15.8 | 18.3 | 6.41 | 6.77 |

Table 6

| species | Beagle dogs | | | | | | | |
|---|---|---|---|---|---|---|---|---|
| Administration Route | Subcutaneous injection | | | | | | Intravenous Injection | |
| Administration Dosage | $3\times10^5$IU/kg | | $6\times10^5$IU/kg | | $1.2\times10^6$IU/kg | | $6\times10^5$IU/kg | |
| Gender | female | male | female | male | female | male | female | male |
| PK parameter (mean, n =3) | | | | | | | | |
| $T_{1/2}$(h) | 6.56 | 6.69 | 4.05 | 5.71 | 5.33 | 7.69 | 9.90 | 8.56 |
| $T_{max}$(h) | 10.7 | 6.67 | 8.00 | 13.3 | 13.3 | 24.0 | - | - |
| $C_{max}$(n g/mL) | 178 | 142 | 611 | 456 | 1183 | 971 | 2240 | 2073 |
| $AUC_{last}$(h*ng/mL) | 5040 | 4334 | 18022 | 15440 | 43473 | 39360 | 33646 | 27584 |
| Vd(mL/kg ) | 120 | 157 | 43.2 | 70.8 | 44.6 | 72.5 | 54.8 | 57.8 |
| CL (mL/h/kg ) | 12.7 | 15.6 | 7.36 | 8.60 | 6.08 | 6.48 | 3.82 | 4.68 |
| $MRT_{last}$(h) | 19.6 | 21.1 | 19.5 | 21.9 | 25.1 | 26.5 | 13.2 | 12.5 |

Pharmacokinetic studies have shown that the half-life of IL-2-HSA in rats and dogs is significantly longer compared with that of natural IL-2 (several minutes in the human body). Thus, the frequency of administration and the total administration dosage can be reduced, the adverse reactions and treatment costs can be decreased, thereby improving the compliance and quality of life of patients.

The above describes the preferred embodiments of the invention in detail. However, the invention is not limited to the specific embodiments. Within the scope of the conception of the invention, various simple modifications to the technical solutions of the invention can be made, and such modifications are also within the scope of protection of the invention.

It should also be indicated that, without contradiction, the specific technical features described in the above specific embodiments can be combined in any appropriate way. To avoid unnecessary duplication, the invention does not further illustrate any possible combinations.

In addition, various embodiments of the invention may also be combined arbitrarily, provided that they do not contravene the conception of the invention. They shall also be regarded as the contents disclosed by the invention.

PCT

Printout (The original is in electronic form)

| 0-1 | Form PCT/RO/134 (SAFE) | |
|---|---|---|
| 0-1-1 | INDICATIONS RELATING TO DEPOSITED MICROORGANISM OR OTHER BIOLOGICAL MATERIAL (PCT Rule 13*bis*) Software version | CEPCT Version 10.28.47 (20220706) MT/FOP 20140331/0.20.5.21 |
| 0-2 | International application No. | PCT/CN2022/106115 |
| 0-3 | Applicant's or agent's file reference | WO11358WDa |

| 1 1-1 1-2 | The indications made below relate to the deposited microorganism or other biological material referred to in the description on page line: | 5 3 |
|---|---|---|
| 1-3 1-3-1 1-3-2 1-3-3 1-3-4 | IDENTIFICATION OF DEPOSIT Name of depositary institution Address of depositary institution Date of deposit Accession Number | China General Microbiological Culture Collection Center Institute of Microbiology Chinese Academy of Sciences, No. 3, Courtyard NO. 1, West Beichen Road, Chaoyang District, Beijing, 100101 May 26, 2022 (26.05.2022) CGMCC 45173 |
| 1-4 | ADDITIONAL INDICATIONS | |
| 1-5 | DESIGNATED STATES FOR WHICH INDICATIONS ARE MADE | All designated states |
| 1-6 | SEPARATE FURNISHING OF INDICATIONS The indications listed below will be submitted to the International Bureau later | |
| | For receiving Office use only | |
| 0-4 | This sheet was received with the international application (Yes or No) | |
| 0-4-1 | Authorized officer | |
| | For International Bureau use only | |
| 0-5 | This sheet was received by the International Bureau on | |
| 0-5-1 | Authorized officer | |

**Claims**

1. A fusion protein of interleukin 2, comprising: a human interleukin 2 or its variant; and human serum albumin or its variant, wherein

the human interleukin 2 or its variants includes: an amino acid sequence as shown in SEQ ID NO: 1; or, an amino acid sequence having at least 90% sequence identity with the amino acid sequence as shown in SEQ ID NO:1;

the human serum albumin or its variants includes: an amino acid sequence as shown in SEQ ID NO:2; or, an amino acid sequence having at least 90% sequence identity with the amino acid sequence shown in SEQ ID NO:2.

2. The fusion protein according to claim 1, wherein the fusion protein includes the amino acid sequence as shown in SEQ ID NO:1 and the amino acid sequence as shown in SEQ ID NO:2;

preferably, the amino acid at position 125 of the amino acid sequence as shown in SEQ ID NO:1 is not cysteine; preferably, the amino acid at position 125 of the amino acid sequence as shown in SEQ ID NO:1 is replaced with serine or alanine.

3. The fusion protein according to claim 1 or 2, wherein the human interleukin 2 or its variant is connected to the human serum albumin or its variant directly or via a linking peptide.

4. The fusion protein according to claim 3, wherein the linking peptide has a general formula of $(G_nS)_m$, in which n or m is an integer selected from 1 to 10; preferably, the linking peptide has a general formula of $(G_nS)_m$, in which n is an integer selected from 1 to 4, and m is an integer selected from 0 to 3.

5. The fusion protein according to claim 1 or 2, wherein the fusion protein has an amino acid sequence as shown in SEQ ID NO:4.

6. An isolated nucleic acid molecule encoding the fusion protein as defined in any one of claims 1 to 5.

7. An expression system comprising a CHO cell which contains the nucleic acid molecule as defined in claim 6,

wherein the expression system expresses the fusion protein as defined in any one of the claims 1 to 5; preferably, the expression system is CHO-K1 cell, which is deposited in China General Microbiological Culture Collection Center on May 26, 2022, under CGMCC No. 45173.

8. A pharmaceutical composition comprising the fusion protein as defined in any one of Claims 1 to 5, and a pharmaceutically acceptable carrier.

9. The pharmaceutical composition according to claim 8, wherein the pharmaceutical composition is in a dosage form of injection, tablet or capsule;

preferably, the dosage form is selected from solution for injection or lyophilized powder for injection; preferably, the carrier is selected from excipient, diluent, filler, binder, wetting agent, disintegrant, absorption enhancer, surfactant, absorptive support, and/or stabilizer.

10. A method for treating or preventing amyotrophic lateral sclerosis, comprising administrating to a subject of an effective amount of the fusion protein as defined in any one of claims 1 to 5, or the pharmaceutical composition as defined in claim 8 or 9.

11. The method according to claim 10, wherein the fusion protein or the pharmaceutical composition is administered orally or by injection, preferably is injected subcutaneously or intravenously.

12. The method according to claim 10 or 11, wherein the fusion protein is administered at $3 \times 10^4$ IU to $1 \times 10^6$ IU each dose; preferably, the pharmaceutical composition is administered at $3 \times 10^4$ IU to $1 \times 10^6$ IU each dose, as measured by the fusion protein therein.

13. The method according to claim 11 or 12, which the fusion protein is administered every 7-28 days, preferably every 14-28 days; preferably, the pharmaceutical composition is administered every 7-28 days, preferably every 14-28 days.

14. Use of the fusion proteins according to claims 1-5 or pharmaceutical compositions according to claims 8 or 9 for the prevention or treatment of amyotrophic lateral sclerosis (ALS), preferably, for the prevention or treatment of decrease in limb strength, body weight, and/or neurological function caused by ALS.

**FIG. 1**

**FIG. 2**

**FIG. 3**

**FIG. 4**

**FIG. 5**

**FIG. 6**

**FIG. 7**

**FIG. 8**

FIG. 9

FIG. 10

IL-2 Stimulate NK-92 Proliferation

**FIG. 11**

IL-2 Stimulate CTLL-2 Proliferation

**FIG. 12**

Time (days)

--- G1 Wild type control
--- G2 Model comparison
--- G3 Edaravone (15mg/kg)
--- G4VDJ010 (100,000 IU/50μl/mouse)

Relative change in weight(g)

1 4 8 11 15 18 22 25 29 32 36 39 44 46 50 53 57 60 64 67 71 74 78 81 85 88 91

**FIG. 13**

ROTAROD TEST

FIG. 14

FIG. 15

FIG. 16

FIG. 17

**FIG. 18**

Kinetic fit — Affinity determination between IL-2-HSA and mIL2R

Kinetic fit — Affinity determination between IL-2-HSA and CaIL2R

Kinetic fit — Affinity determination between IL-2-HSA and hIL2R

Kinetic fit — Affinity determination between IL-2-HSA and rIL2R

FIG. 19

Day 3 Comparison of the effects of IL-2-HSA and rhIL-2 on in vitro proliferation human peripheral blood $T_{reg}$ cells

**FIG. 20**

**FIG. 21**

## INTERNATIONAL SEARCH REPORT

| International application No. |
|---|
| **PCT/CN2022/106115** |

**A. CLASSIFICATION OF SUBJECT MATTER**

C12N 15/85(2006.01)i; C07K 19/00(2006.01)i; C12N 15/62(2006.01)i; A61K 38/20(2006.01)i; A61K 47/64(2017.01)i; A61P 25/14(2006.01)i

According to International Patent Classification (IPC) or to both national classification and IPC

**B. FIELDS SEARCHED**

Minimum documentation searched (classification system followed by classification symbols)

C12N, C07K, A61K, A61P

Documentation searched other than minimum documentation to the extent that such documents are included in the fields searched

Electronic data base consulted during the international search (name of data base and, where practicable, search terms used)

CNMED, CNABS, DWPI, WPABS, WPABSC, CJFD, AUABS, TWMED, ILABS, HKABS, MOABS, CNTXT, ENTXT, ENTXTC, WOTXT, USTXT, VCN, VEN, GBTXT, EPTXT, CATXT, CNKI, PubMed, EMBL, Web of Science, GoogleScholar: 白细胞介素2, 人血清白蛋白, 肌萎缩侧索硬化症 (Amyotrophic lateral sclerosis, ALS), C125A, Interleukin-2(IL-2), Human Serum Albumin (HSA) 中国专利生物序列检索系统, Chinese Patent Biological Sequence Retrieval System, NCBI, EMBL: 基于SEQ ID NO: 1, 2, 4的序列检索, Sequence search based on SEQ ID NOs: 1, 2, and 4

**C. DOCUMENTS CONSIDERED TO BE RELEVANT**

| Category* | Citation of document, with indication, where appropriate, of the relevant passages | Relevant to claim No. |
|---|---|---|
| PX | CN 113789346 A (BEIJING VDJBIO CO., LTD.) 14 December 2021 (2021-12-14) claims 1-10 | 1-8 |
| X | CN 113789346 A (BEIJING VDJBIO CO., LTD.) 14 December 2021 (2021-12-14) claims 1-10 | 9 |
| Y | CN 113789346 A (BEIJING VDJBIO CO., LTD.) 14 December 2021 (2021-12-14) claims 1-10 | 10-14 |
| X | CN 112724259 A (SINOBIOTECH (TIANJIN) LTD.; FORTUNEROCK (CHINA) CO., LTD.; TIANJIN SINOBIOTECH LTD.; BEIJING BIO-FORNTUNE LTD.) 30 April 2021 (2021-04-30) claims 1, 5, and 6, sequences 9 and 10, and description, paragraph 160 | 1-9 |
| Y | CN 112724259 A (SINOBIOTECH (TIANJIN) LTD.; FORTUNEROCK (CHINA) CO., LTD.; TIANJIN SINOBIOTECH LTD.; BEIJING BIO-FORNTUNE LTD.) 30 April 2021 (2021-04-30) claims 1, 5, and 6, sequences 9 and 10, and description, paragraph 160 | 10-14 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 September 2022** | **13 October 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/106115** |

**C.   DOCUMENTS CONSIDERED TO BE RELEVANT**

| Category* | Citation of document, with indication, where appropriate, of the relevant passages | Relevant to claim No. |
|---|---|---|
| Y | GIOVANNELLI, I. et al. "Amyotrophic Lateral Sclerosis Transcriptomics Reveals Immunological Effects of Low-dose Interleukin-2" *Brain Communications*, Vol. 3, No. 3, 29 June 2021 (2021-06-29), abstract | 10-14 |
| X | CN 1626554 A (INSTITUTE OF BIOTECHNOLOGY, ACADEMY OF MILITARY MEDICAL SCIENCES OF PLA) 15 June 2005 (2005-06-15) see abstract | 1-9 |
| Y | CN 1626554 A (INSTITUTE OF BIOTECHNOLOGY, ACADEMY OF MILITARY MEDICAL SCIENCES OF PLA) 15 June 2005 (2005-06-15) see abstract | 10-14 |
| A | US 2018030106 A1 (INDUSTRY ACADEMY COOPERATION FOUNDATION OF SOONCHUNHYANG UNIVERSITY) 01 February 2018 (2018-02-01) see abstract | 1-14 |

Form PCT/ISA/210 (second sheet) (January 2015)

**INTERNATIONAL SEARCH REPORT**

International application No.

**PCT/CN2022/106115**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] The actually submitted sequence table is an XML file of the Standard ST.26.

Form PCT/ISA/210 (continuation of first sheet) (January 2015)

## INTERNATIONAL SEARCH REPORT

International application No.

**PCT/CN2022/106115**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **10-14**
   because they relate to subject matter not required to be searched by this Authority, namely:

   [1] Claims 10-14 relate to a method for treating or preventing amyotrophic lateral sclerosis, falling within the scope of methods for treating a human or animal body, and belonging to the cases listed in PCT Rule 39.1(iv) for which no international search is required. Therefore, no international search is conducted. A search was conducted on the basis of a use of the fusion protein according to claims 1-5 or the pharmaceutical composition according to claim 8 or 9 in the preparation of a drug for treating or preventing amyotrophic lateral sclerosis.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

| International application No. |
|---|
| **PCT/CN2022/106115** |

| Patent document cited in search report | | | Publication date (day/month/year) | Patent family member(s) | | | Publication date (day/month/year) |
|---|---|---|---|---|---|---|---|
| CN | 113789346 | A | 14 December 2021 | None | | | |
| CN | 112724259 | A | 30 April 2021 | None | | | |
| CN | 1626554 | A | 15 June 2005 | None | | | |
| US | 2018030106 | A1 | 01 February 2018 | WO | 2016068427 | A1 | 06 May 2016 |
| | | | | WO | 2016068428 | A1 | 06 May 2016 |
| | | | | US | 2018237488 | A1 | 23 August 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022106115 W

**Non-patent literature cited in the description**

- Molecular Cloning: A Laboratory manual. Cold Spring Harbor Laboratory Press